# EUROPEAN PATENT APPLICATION

(11) **EP 4 538 348 A1**
(43) Date of publication of application: **16.04.2025**
(21) Application number: 23202533.8
(22) Date of filing: 09.10.2023
(51) Int. Cl.: C09K 11/06, G01N 21/64, G01N 33/58, C07D 221/14

(54) **LUMINESCENT COMPOUND AND COMPOSITION, METHOD AND USE RELATED THERETO**

(71) Applicant: The Provost, Fellows, Foundation Scholars, & the other members of Board, of the College of the Holy & Undiv. Trinity of Queen Elizabeth near Dublin, Dublin 2 (IE)
(72) Inventor: HENWOOD, Adam Francis, c/o Trinity College Dublin, College Green, Dublin 2 (IE); GUNNLAUGSSON, Thorfinnur, c/o Trinity College Dublin, College Green, Dublin 2 (IE); O'SHEA, Donal Finbar, c/o Royal College Of Surgeons in Ireland, Dublin 2 (IE); SIGURVINSSON, Louisa Constance, c/o Trinity College Dublin, College Green, Dublin 2 (IE)
(74) Representative: Appleyard Lees IP LLP

(57) **Abstract**

A method of imaging a cellular composition is disclosed. The method comprises the steps of contacting the cellular composition with a luminescent compound and imaging the cellular composition with a time-resolved spectroscopic technique. The luminescent compound exhibits a first luminescent state having a first emission lifetime in a first chemical environment and a second luminescent state having a second, different, emission lifetime in a second chemical environment. Also disclosed are luminescent compounds, compositions comprising an amphiphilic polymer and a luminescent compound, and the use of a luminescent compound in combination with a time-resolved spectroscopic technique for imaging a cellular composition.

## Description

### Field of the Invention

The present invention relates to a luminescent compound, a composition comprising a luminescent compound, a method of imaging a cellular composition involving a luminescent compound, and the use of a luminescent compound for imaging a cellular composition. In particular the invention relates to a luminescent compound exhibiting multiple different luminescent states according to the chemical environment of the compound, said luminescent states being distinguishable by a time-resolved spectroscopic technique.

### Background

Aggregation-induced emission (AIE) is a luminescence phenomenon characterised by large changes in the emission intensities of luminophores as a function of their solvation states: upon dissolution in good solvent media the AIE molecules are non-emissive but aggregating them together in poor solvents or in the solid state causes them to produce bright luminescence instead. Such an evolution in the properties of the aggregates as a function of their size, which is not necessarily programmed into the individual component molecules, is a strictly emergent phenomenon.

A large number of mechanisms for AIE are known. While the details of these mechanisms can differ significantly, broadly speaking they are almost always underpinned by suppressing the population of inherently non-emissive states to promote population of emissive excited states via aggregation. Examples include: arresting deleterious free rotations of aromatic units in solution by conformationally locking them in an aggregated state; stabilising the energy of emissive electronic states relative to non-emissive electronic states; and vibrations blocking photochemical reactions from forming AIE inactive products. The AlE molecules currently known almost always manifest the same optical "turn-off/turn-on" switching phenomena. Accordingly, these systems are almost exclusively studied under distinctly thermodynamic conditions using techniques like steady-state emission spectroscopy and confocal microscopy, particularly with AIE systems designed for bioimaging.

In recent years it has been found that the spectroscopic signatures of the lifetimes of AIE dyes, rather than their emission profiles, may be used to obtain information about the dyes' environments via time-resolved fluorescence lifetime imaging (FLIM). Such examples are still limited and they mostly consist of aggregation-induced delayed fluorescence (AIDF) compounds that exhibit excited state lifetimes on the order of microseconds, which opens up time gating for time-resolved cellular imaging applications.

There have been two AlE studies in which specific properties within a subcellular environment were mapped as a function of changes in lifetime of the luminophore. In one, hexaphenylsilole (HPS) was used to monitor local fluctuations in temperature (Gao, H., Kam, C., Chou, T. Y., Wu, M.-Y., Zhao, X. and Chen, S., (2020). A simple yet effective AlE-based fluorescent nano-thermometer for temperature mapping in living cells using fluorescence lifetime imaging microscopy. Nanoscale Horiz. 5, 488-494). In the other, a pH-sensitive, tetraphenylethylene-hemicyanine (TPE-Cy) conjugate was used as the reporter molecule for mapping cellular viscosity (Chen, S., Hong, Y., Zeng, Y., Sun, Q., Liu, Y., Zhao, E., Bai, G., Qu, J., Hao, J. and Tang, B. Z., (2015). Mapping live cell viscosity with an aggregation-induced emission fluorogen by means of two-photon fluorescence lifetime imaging. Chem. Eur. J. 21, 4315-4320). These examples nevertheless represent the same emergent, turn-off/turn-on paradigm described above.

There remains a need for luminescent compounds which can be used to report information about their environments.

### Summary of the Invention

According to a first aspect of the present invention, there is provided a method of imaging a cellular composition comprising the steps of:
contacting the cellular composition with a luminescent compound that exhibits a first luminescent state having a first emission lifetime in a first chemical environment and a second luminescent state having a second emission lifetime in a second chemical environment, wherein the first emission lifetime is different to the second emission lifetime; and
imaging the cellular composition with a time-resolved spectroscopic technique.

According to a second aspect of the present invention, there is provided a use of a luminescent compound in combination with a time-resolved spectroscopic technique for imaging a cellular composition, wherein the compound exhibits a first luminescent state having a first emission lifetime in a first chemical environment and a second luminescent state having a second emission lifetime in a second chemical environment, wherein the first emission lifetime is different to the second emission lifetime.

The inventors have found that luminescent compounds having multiple luminescent states depending on the chemical environment of the compound may advantageously be used for imaging a cellular composition. Cellular compositions typically comprise a number of different chemical environments, such as lipid droplets or aqueous extracellular environments, and thus the luminescent compound will exhibit a different luminescent state according to its location in the cellular composition. Furthermore, since the luminescent states of the compound have different emission lifetimes, a time-resolved spectroscopic technique may advantageously be used to distinguish between the luminescent states (and therefore, the chemical environment) even if there is substantial overlap between the emission wavelengths of the luminescent states.

Suitable features of the first and second aspects will now be described.

By "cellular composition" is meant a composition comprising one or more cells or parts thereof. In some embodiments, cells are absent from the cellular composition and the cellular composition comprises parts of cells, such as organelles. Preferably, the cellular composition comprises cells. The cells may comprise live cells. The cells may comprise cancer cells. suitably, the cellular composition is outside of a human or animal body. The method and use of the first and second aspects are suitably in vitro.

By "luminescent state" is meant an excited state in which the compound emits electromagnetic radiation, such as visible light.

By "emission lifetime" is meant the excited state lifetime, which corresponds to the time elapsed between the start and the end of the emission of electromagnetic radiation, suitably following exposure to excitation energy. The emission may have a single component (indicated by monoexponential decay) or multiple components (indicated by multi-exponential decay), such as two components. When the emission has multiple components, the emission lifetime is taken to encompass all components of the emission.

Suitably, at least a portion of the electromagnetic radiation emitted by compound in the first luminescent state is visible light. Suitably, at least a portion of the electromagnetic radiation emitted by the compound in the second luminescent state is visible light. Preferably, at least a portion of the electromagnetic radiation emitted by compound in the first luminescent state is visible light and at least a portion of the electromagnetic radiation emitted by the compound in the second luminescent state is visible light. Suitably, the electromagnetic radiation emitted by the compound in the first luminescent state has an emission maximum in the visible spectrum. Suitably, the electromagnetic radiation emitted by the compound in the second luminescent state has an emission maximum in the visible spectrum. Preferably, the electromagnetic radiation emitted by the compound in the first luminescent state has an emission maximum in the visible spectrum and the electromagnetic radiation emitted by the compound in the second luminescent state has an emission maximum in the visible spectrum.

By "visible light" is meant electromagnetic radiation having typically a wavelength in the range of 380 to 700 nm. "Visible spectrum" refers to the range of 380 to 700 nm.

The luminescent compound exhibits the first luminescent state in a first chemical environment and the second luminescent state in a second chemical environment. Suitably, the luminescent states are attained by exposing the luminescent compound to excitation energy, such as electromagnetic radiation, heat, or mechanical energy. The method of the first aspect may comprise the step of exposing the luminescent compound to excitation energy. Said step is suitably carried out before or simultaneously with the step of imaging the cellular composition. Preferably, the excitation energy is electromagnetic radiation, such as ultraviolet radiation or visible light. The electromagnetic radiation may be in the form of a laser. Suitably, the luminescent compound exhibits the first luminescent state when in a first chemical environment and exposed to excitation energy and the luminescent compound exhibits the second luminescent state when in a second chemical environment and exposed to excitation energy. Suitably, the same excitation energy is used to excite the luminescent compound into the first luminescent state and the second luminescent state.

By "ultraviolet radiation" is meant electromagnetic radiation having typically a wavelength in the range of 10 to 379 nm.

Preferably, the luminescent compound is a fluorescent compound, the first luminescent state is a first fluorescent state, and the second luminescent state is a second fluorescent state. The fluorescent states are suitably attained by exposing the fluorescent compound to ultraviolet radiation and/or visible light. The ultraviolet radiation and/or visible light suitably has a wavelength in the range of 350 to 400 nm. Ultraviolet radiation is preferred. The ultraviolet radiation and/or visible light may be in the form of a laser. Suitably, the ultraviolet radiation or the visible light has a single wavelength. The method of the first aspect may comprise the step of exposing the fluorescent compound to ultraviolet radiation and/or visible light, preferably ultraviolet radiation. Said step is suitably carried out before or simultaneously with the step of imaging the cellular composition. Suitably, the fluorescent compound exhibits the first fluorescent state when in a first chemical environment and exposed to ultraviolet radiation and/or visible light and the fluorescent compound exhibits the second fluorescent state when in a second chemical environment and exposed to ultraviolet radiation and/or visible light. Suitably the same ultraviolet radiation and/or visible light is used to excite the fluorescent compound into the first fluorescent state and the second fluorescent state.

The term "chemical environment" will be well-known by the skilled person. A "chemical environment" as defined herein suitably refers to the molecules, functional groups, or mixtures thereof which form the environment around the luminescent compound. The first chemical environment is different to the second chemical environment. The first chemical environment and second chemical environment therefore suitably contain different molecules, functional groups, or mixtures thereof. By way of example, a first environment and a second environment which differ only in physical properties such as temperature or viscosity do not correspond to a first chemical environment and a second chemical environment as defined herein.

The luminescent compound suitably exhibits a non-luminescent state in a third chemical environment. The third chemical environment is different to the first chemical environment and the second chemical environment. In the non-luminescent state, the luminescent compound suitably emits no electromagnetic radiation even when exposed to excitation energy, or it emits electromagnetic radiation at an intensity that is less than 20%, preferably less than 10%, such as less than 5% the intensity of the electromagnetic radiation of either of the first luminescent state or the second luminescent state.

The luminescent compound is suitably non-aggregated in the first chemical environment. The first chemical environment may be an intracellular environment, such as a lipid droplet. The first chemical environment may be a first solvent medium. The first solvent medium may comprise one or more solvents.

The luminescent compound is suitably aggregated in the second chemical environment. The second luminescent state suitably corresponds to aggregation-induced emission (AIE). The second chemical environment may be an extracellular environment. The second chemical environment may be a second solvent medium. The second solvent medium may comprise one or more solvents.

The luminescent compound is suitably non-aggregated in the third chemical environment. The third chemical environment may be a third solvent medium. The third solvent medium may comprise one or more solvents. Preferably, the third solvent medium is a mixture of the first solvent medium and the second solvent medium. The ratio of the first solvent medium and the second solvent medium will depend on the identity of the compound. In some embodiments, the third solvent medium is a mixture of the first solvent medium and the second solvent medium in a ratio of from 1:99 to 20:80 by volume, such as from 5:95 to 30:70 by volume, for example from 10:90 to 50:50 by volume. The non-luminescence of the luminescent compound in a mixture of the first solvent medium and the second solvent medium is advantageous because it facilitates identification of the first luminescent state and the second luminescent state and therefore facilitates identification of distinct chemical environments in the cellular composition.

The first solvent medium, second solvent medium, and third solvent medium are not necessarily present in the cellular composition. Rather, the solvent media may be used to test whether the luminescent compound exhibits the first luminescent state and the second luminescent state. The solvent media may act as approximations of chemical environments present in the cellular composition.

The luminescent compound is suitably soluble in the first solvent medium. The luminescent compound is suitably insoluble in the second solvent medium. The luminescent compound is suitably at least partially soluble in the third solvent medium. The first solvent medium may be miscible with the second solvent medium. The insolubility of the luminescent compound in the second solvent medium suitably causes the luminescent compound to aggregate in the second solvent medium.

The polarity of the first solvent medium is suitably lower than the polarity of the second solvent medium. The polarity of the first solvent medium is suitably lower than the polarity of the third solvent medium. The polarity of the second solvent medium is suitably higher than the polarity of the third solvent medium. Suitably, the polarity of the first solvent medium is low enough to allow the luminescent compound to exhibit the first luminescent state. This is typically due to solvatochromic and energy gap law effects. The third solvent medium typically has a polarity which is neither low enough to allow the luminescent compound to exhibit the first luminescent state nor high enough for the luminescent compound to aggregate and exhibit the second luminescent state. The first solvent medium may comprise one or more solvents having a relative polarity of less than 0.500, such as less than 0.400, for example less than 0.300. In some embodiments, the first solvent medium does not comprise solvents having a relative polarity of at least 0.500, such as at least 0.400, for example at least 0.300. The relative polarity of solvents is disclosed in Christian Reichardt and Thomas Welton, Solvents and Solvent Effects in Organic Chemistry, Wiley-VCH Publishers, 4th edition, 2011.

The first solvent medium may comprise a solvent selected from acetone, dioxane, isopropanol, pyridine, tetrahydrofuran (THF), and mixtures thereof. Preferably, the first solvent medium comprises tetrahydrofuran. In some embodiments, the first solvent medium consists of tetrahydrofuran.

The second solvent medium may comprise one or more solvents having a relative polarity of at least 0.500, such as at least 0.600, for example at least 0.700. In some embodiments, the second solvent medium does not comprise solvents having a relative polarity of less than 0.500, such as less than 0.600, for example less than 0.700. Alternatively, the second solvent medium may comprise a mixture of one or more solvents having a relative polarity of at least 0.500 and one or more solvents having a relative polarity of less than 0.500, such as a mixture of one or more solvents having a relative polarity of at least 0.600 and one or more solvents having a relative polarity of less than 0.400, for example a mixture of one or more solvents having a relative polarity of at least 0.700 and one or more solvents having a relative polarity of less than 0.300.

Preferably, the second solvent medium comprises water. In some embodiments, the second solvent medium consists of water. Alternatively, the second solvent medium comprises a mixture of water and one or more solvents selected from acetone, dioxane, isopropanol, pyridine, and tetrahydrofuran. Suitably, the second solvent medium comprises water in an amount of at least 50%, such as at least 60%, such as at least 70%, such as at least 80%, for example at least 90% based on the total volume of the second solvent medium.

The third solvent medium may comprise water, for example when the third solvent medium is a mixture of the first solvent medium and the second solvent medium. Suitably, the third solvent medium comprises water in an amount of less than 90%, such as less than 80%, such as less than 70%, such as less than 60%, for example less than 50% based on the total volume of the third solvent medium.

Suitably, the first emission lifetime has a single component. Suitably, the second emission lifetime has a first component and a second component. The two components of the second emission lifetime are believed to be due to the aggregation of the luminescent compound in the second chemical environment, which suppresses the movement of the luminescent compound, such as the rotation of aromatic groups.

The second emission lifetime is suitably longer than the first emission lifetime. The second emission lifetime may be at least 10% longer, such at least 25% longer, for example at least 50% longer than the first emission lifetime. In some embodiments, the second emission lifetime may be at least 1 ns longer, such at least 3 ns longer, for example at least 10 ns longer than the first emission lifetime.

The luminescent compound suitably comprises organic moieties. Suitably, the luminescent compound is an organic, organometallic, or coordination compound. Preferably, the luminescent compound is an organic compound, especially a fluorescent organic compound.

By "organic" compounds or moieties is meant compounds or moieties that comprise carbon, but which do not comprise metals. By "organometallic" is meant compounds or moieties that comprise carbon and at least one metal and at least one metal-carbon bond. By "coordination compound" is meant a metal complex not comprising any metal-carbon bonds. Coordination compounds as used herein preferably comprise an organic moiety.

The luminescent compound suitably comprises a plurality of freely rotating aromatic groups, preferably at least three, such as at least four. The presence of a plurality of freely rotating aromatic groups in the luminescent compound may advantageously increase the intensity of the second luminescent state.

The plurality of freely rotating aromatic groups may comprise at least two, preferably at least three, such as at least four freely rotating aromatic groups.

Each freely rotating aromatic group may comprise one or more aromatic rings. Each freely rotating aromatic group may be a monocyclic aromatic group or a polycyclic aromatic group. Each freely rotating aromatic group may comprise one or more aromatic heteroatoms, such as oxygen, nitrogen, and/or sulfur.

Preferably, the freely rotating aromatic groups together form a conjugated system. Suitably, each of the freely rotating aromatic groups is linked to at least one of the other freely rotating aromatic groups by a single bond.

The luminescent compound may be represented by formula (I):

A-X-B (I)

wherein A is an electron acceptor moiety, X is a bond or a linker moiety, and B is an electron donor moiety.

Suitably, A comprises an aromatic group. The aromatic group may be a monocyclic aromatic group or a polycyclic aromatic group. The aromatic group may comprise one or more aromatic heteroatoms, such as oxygen, nitrogen, or sulfur. Suitably, the aromatic group is a benzene, naphthalene or anthracene group. Preferably, the aromatic group is a naphthalene group.

The aromatic group is suitably substituted with one or more electron-withdrawing groups. Suitable electron-withdrawing groups include nitro, cyano, sulfonate, and carbonyl groups (including ester groups, anhydride groups, amide groups and imide groups). Preferably, the aromatic group is substituted with an imide group. The imide group is suitably a cyclic imide group fused to the aromatic group.

Preferably, A comprises a phthalimide group, a naphthalimide group, or an anthraceneimide group.

A may have the structure: 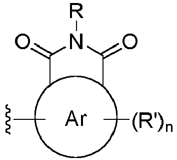
wherein R is an organic or organometallic group or a metal complex;
Ar is a monocyclic aromatic group or a polycyclic aromatic group;
n is 0, 1, 2, 3, or 4; and
each R' is independently an amino group or a hydrocarbyl group, wherein the hydrocarbyl group is optionally interrupted by an oxygen atom or a sulfur atom.

By "optionally interrupted" is meant that an oxygen or sulfur atom may be inserted in a carbon-carbon bond of the hydrocarbyl group so as to form an ether or thioether moiety.

Suitably, each R' is independently an amino group or an alkyl, alkenyl, or aryl group, wherein the alkyl or alkenyl group is optionally interrupted by an oxygen atom or a sulfur atom. Preferably, each R' is independently an amino group or an alkyl group, wherein the alkyl group is optionally interrupted by an oxygen atom or a sulfur atom. The alkyl group suitably has from 1 to 6 carbon atoms. In some embodiments, each R' is independent methyl or ethyl. Suitably, n is 0 or 1. Preferably, n is 0.

A suitably has the structure:

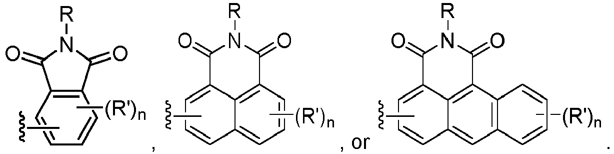

For the avoidance of doubt, the undefined bonds in these structures (i.e. the bond to X and bonds to any R' groups) may be to any possible carbon atom in the monocyclic or polycyclic aromatic rings.

A preferably has the structure:

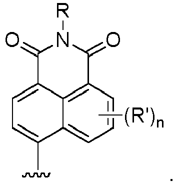

A further preferably has the structure: 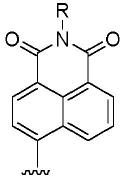 wherein R is an organic or organometallic group or a metal complex.

The identity of R does not generally affect the photophysical properties of the luminescent compound. The skilled person will therefore understand that R may be any suitable substituent.

Suitably, R is an optionally substituted hydrocarbyl group. R may be an optionally substituted alkyl, alkenyl, alkynyl, aryl, or heteroaryl group. Preferably, R is an optionally substituted alkyl, aryl, or heteroaryl group.

Any suitable substituents may be selected. The optional substituents of R may be selected from alkyl, alkenyl, alkynyl, aryl, heteroaryl, hydroxyl, amino, cyano, alkoxy, carboxyl, ester, amide, ammonium, and phosphonium groups, metal complexes, and biological moieties, each of which are optionally substituted. The biological moieties may comprise sugars or lipids such as cholesterol.

R may be an optionally substituted alkyl group. The alkyl group may be straight-chain or branched. The alkyl group suitably has from 1 to 30 carbon atoms, such as from 1 to 12 carbon atoms, for example from 1 to 8 carbon atoms. R may be an unsubstituted alkyl group suitably having from 1 to 30 carbon atoms, such as from 1 to 12 carbon atoms, for example from 1 to 8 carbon atoms. In some embodiment R is an octyl group. R may be a substituted alkyl group. The alkyl group is suitably substituted with a heteroaryl, amino, alkoxy, carboxyl, amide, or phosphonium group, or a biological moiety, each of which are optionally substituted.

R may be an optionally substituted aryl group. The aryl group is preferably phenyl. R may be an unsubstituted aryl group. R may be a substituted aryl group. The aryl group is suitably substituted with an optionally substituted aryl group.

R may be an optionally substituted heteroaryl group. The heteroaryl group may comprise oxygen, nitrogen, and/or sulfur. The heteroaryl group is preferably pyridyl, especially 4-pyridyl. R may be an unsubstituted heteroaryl group. R may be a substituted heteroaryl group. The heteroaryl group is suitably substituted with a metal complex. The metal complex is suitably a rhenium complex.

In some preferred embodiments, R is selected from:

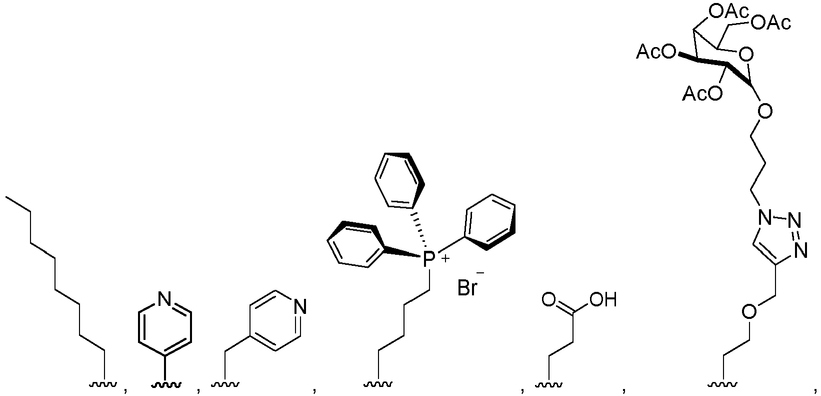

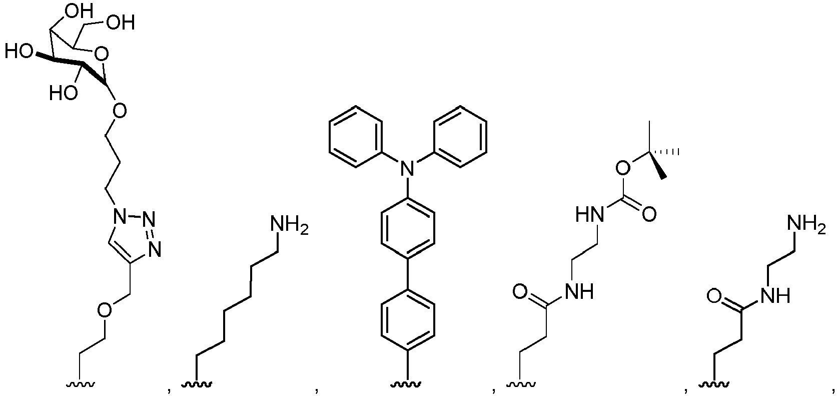

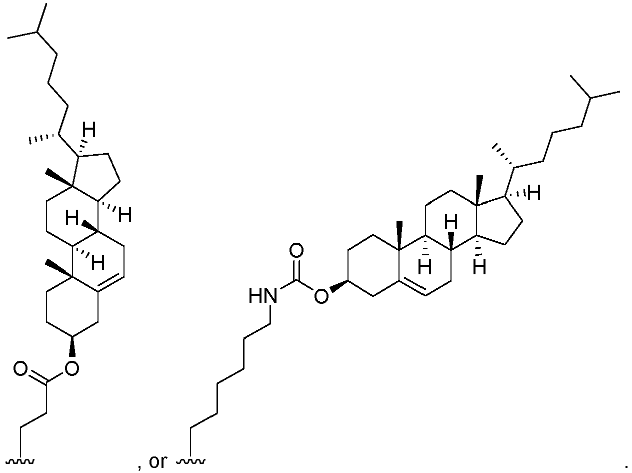

In some embodiments, R is a multivalent group and the luminescent compound has the structure: 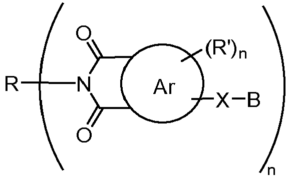 wherein n is at least 2.

In such embodiments, the luminescent compound suitably has the structure:

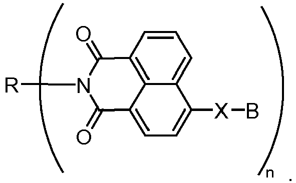

Suitably, n is 2, 3. Or 4. Preferably, n is 3.

In some preferred embodiments, n is 3 and R has the structure:

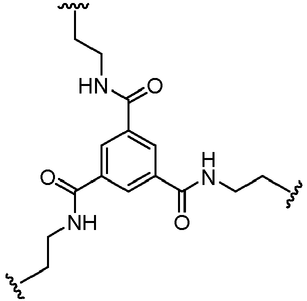

In some embodiments, X is a bond and the luminescent compound may be represented by formula (II):

A-B (II)

wherein A and B are as defined herein.

Preferably, X is a linker moiety. Suitably, X comprises an aromatic group. X may comprise a monocyclic aromatic group or a polycyclic aromatic group.

X may have the structure: 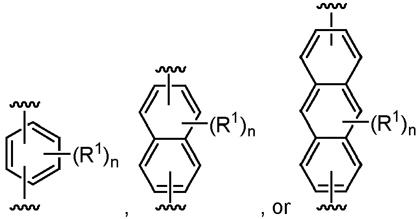 wherein n is 0, 1, 2, 3, or 4 and each R¹ is independently a hydrocarbyl group. Suitably, each R¹ is independently an alkyl, alkenyl, or aryl group. Preferably, each R¹ is independently an alkyl group. The alkyl group suitably has from 1 to 6 carbon atoms. In some embodiments, each R¹ is independent methyl or ethyl. Suitably, n is 0 or 1. Preferably, n is 0.

For the avoidance of doubt, the undefined bonds in these structures (i.e. the bond to A or B and bonds to any R¹ groups) may be to any possible carbon atom in the monocyclic or polycyclic aromatic rings.

Suitably, X has the structure:

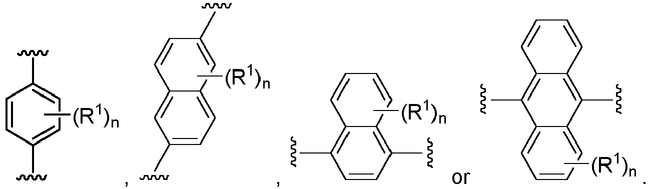

Preferably, X has the structure:

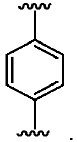

Suitably, B comprises an aromatic group. the aromatic group may be a monocyclic aromatic group or a polycyclic aromatic group. The aromatic group may comprise one or more aromatic heteroatoms, such as oxygen, nitrogen, or sulfur.

The aromatic group is suitably substituted with one or more electron-donating groups. Suitable electron-donating groups include optionally substituted alkyl, aryl, hydroxy, amino, and alkoxy groups.

B may be selected from: 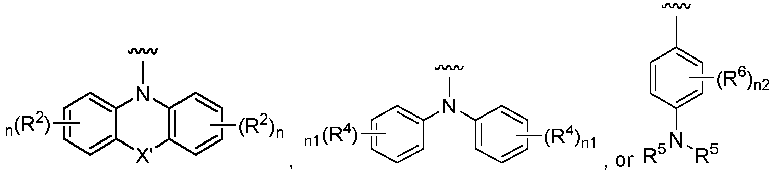
wherein each n is independently 0, 1, 2, 3, or 4;
each R² is independently an electron-donating group;
X' is a bond, oxygen, NH, NR³, sulfur, or an alkylene group;
R³ is a hydrocarbyl group;
each n1 is independently 0, 1, 2, 3, 4, or 5;
each R⁴ is independently an electron-donating group;
n2 is 0, 1, 2, 3 or 4;
each R⁵ is independently a hydrocarbyl group; and
each R⁶ is independently an electron-donating group; or
each R⁵ may independently be taken together with an R⁶ group and the intervening atoms to form a heterocycle.

B may have the structure: 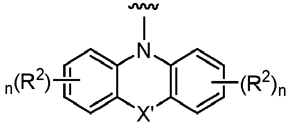
wherein each n is independently 0 or 1;
each R² is independently an optionally substituted alkyl, aryl, hydroxy, amino, or alkoxy group;
X' is a bond, oxygen, NH, NR³, sulfur, or a methylene group optionally substituted with one or two alkyl groups; and
R³ is an alkyl, alkenyl, or aryl group;

B may have the structure: 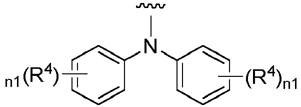
wherein each n1 is independently 0 or 1; and
each R⁴ is independently an optionally substituted alkyl, aryl, hydroxy, amino, or alkoxy group.

B may have the structure: 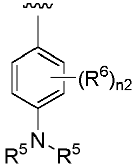
wherein n2 is 0, 1, 2, 3 or 4;
each R⁵ is independently an alkyl, alkenyl, or aryl group; and
each R⁶ is independently an optionally substituted alkyl, aryl, hydroxy, amino, or alkoxy group; or
each R⁵ may independently be taken together with an R⁶ group and the intervening atoms to form a 5 to 7-membered heterocycle.

Suitably, B is selected from: 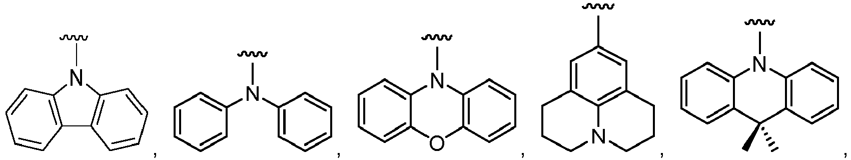 or

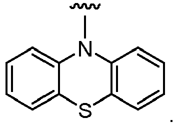

Preferably, B is selected from:

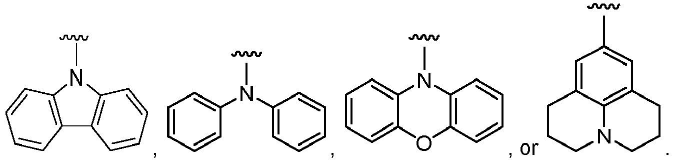

Further preferably, B is selected from:

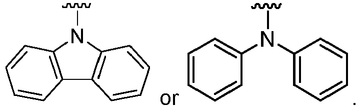

In some preferred embodiments, the luminescent compound is represented by formula (I):

A-X-B (I)

wherein A has the structure: 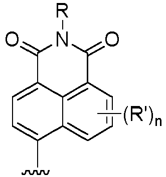
wherein R is an organic or organometallic group or a metal complex, n is 0, 1, 2, 3, or 4, and each R' is independently a hydrocarbyl group;
X is a bond or has the structure: 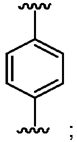 and
B is selected from: 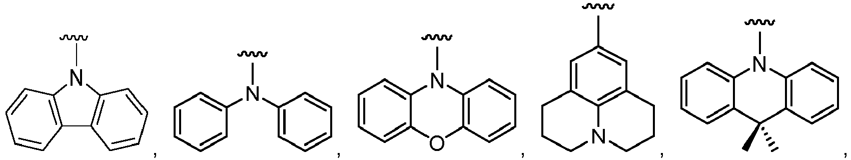 or

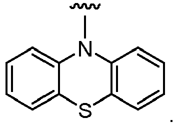

In some preferred embodiments, the luminescent compound is represented by formula (I):

A-X-B (I)

wherein A has the structure: 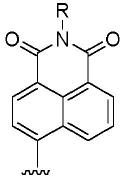
wherein R is an organic or organometallic group or a metal complex;
X has the structure: 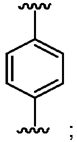 and
B is selected from:

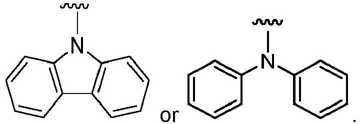

In some preferred embodiments, the luminescent compound is represented by formula (II):

A-B (II)

wherein A has the structure: 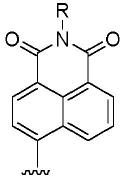
wherein R is an organic or organometallic group or a metal complex; and
B is selected from:

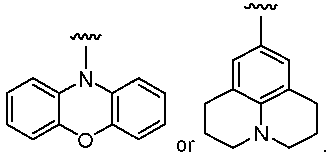

In some preferred embodiments, the luminescent compound is selected from one of compounds 1 to 22:

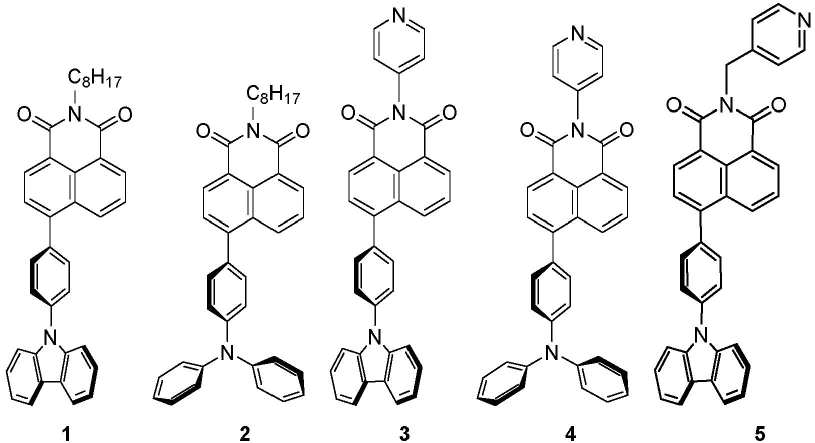

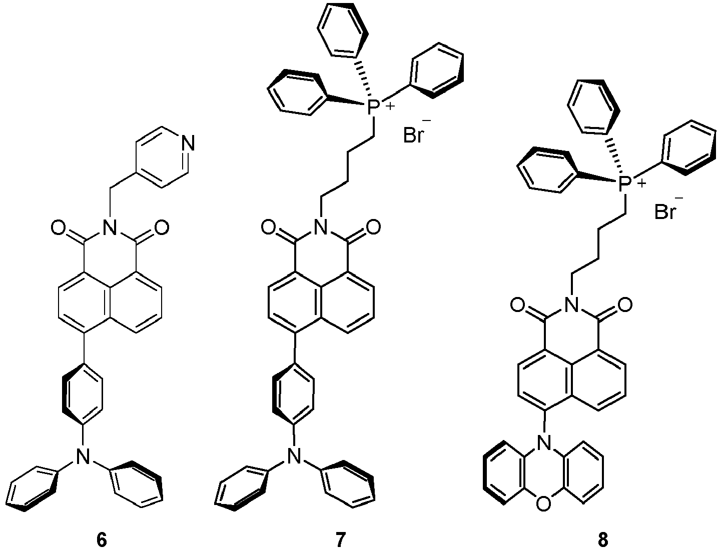

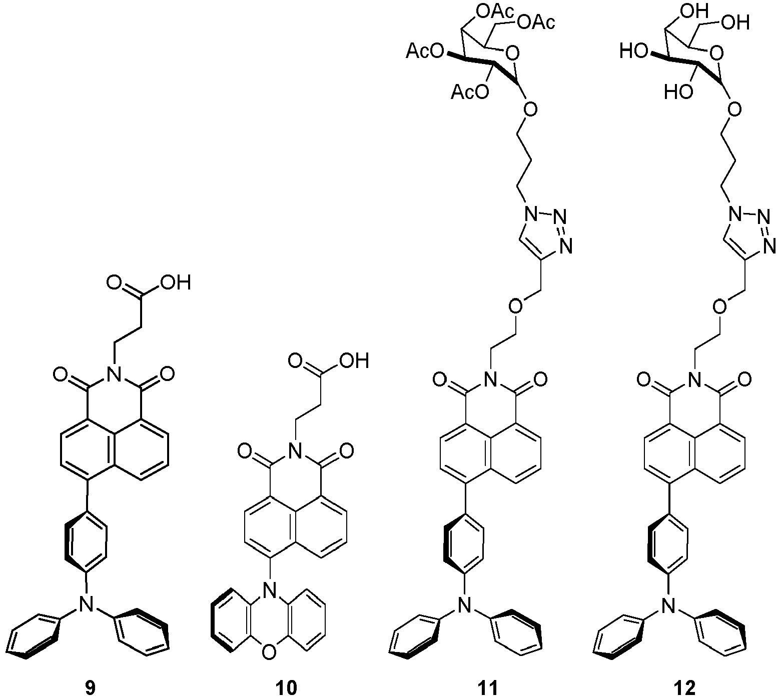

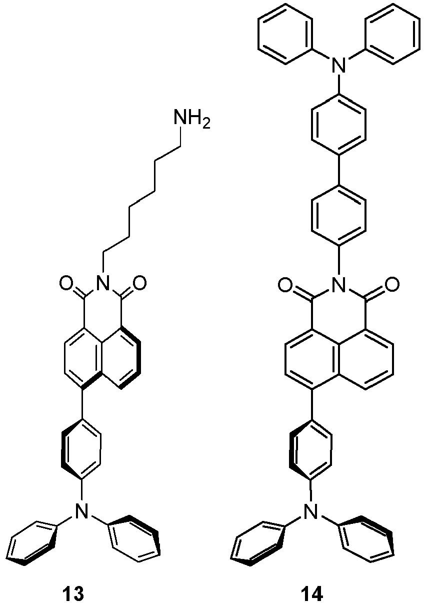

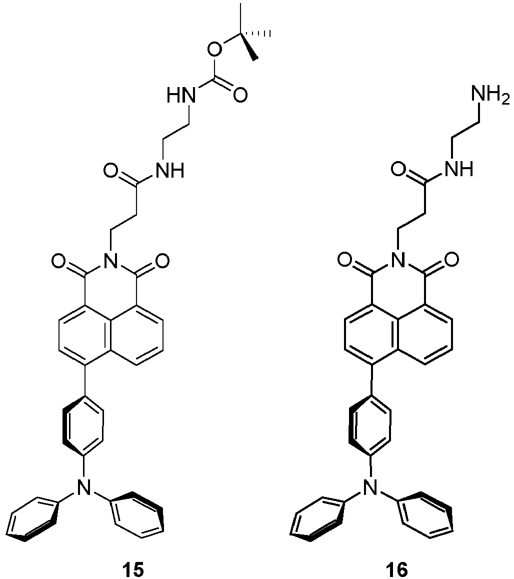

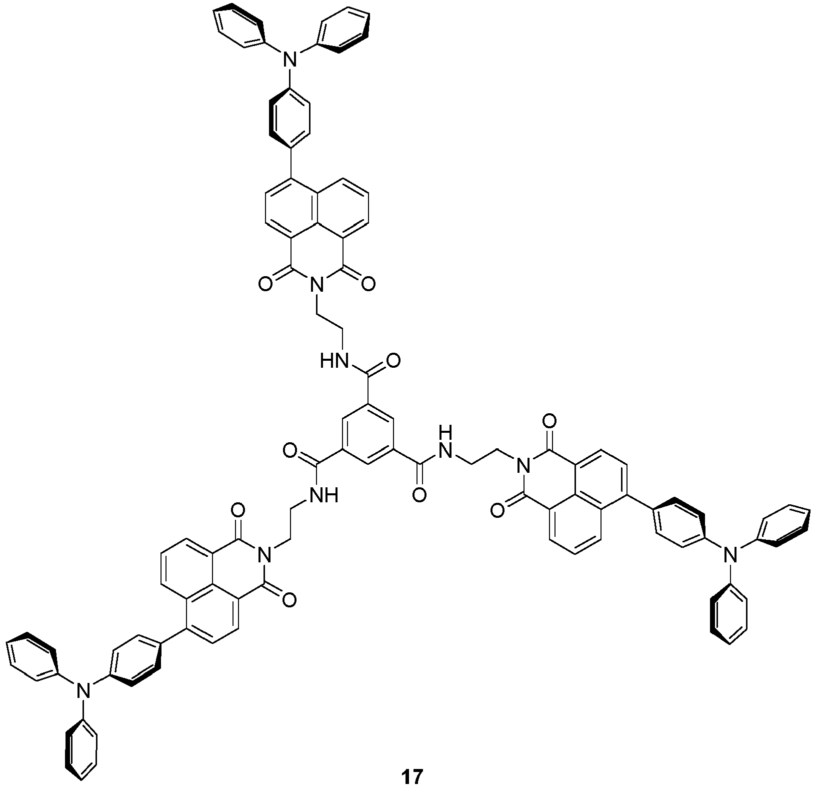

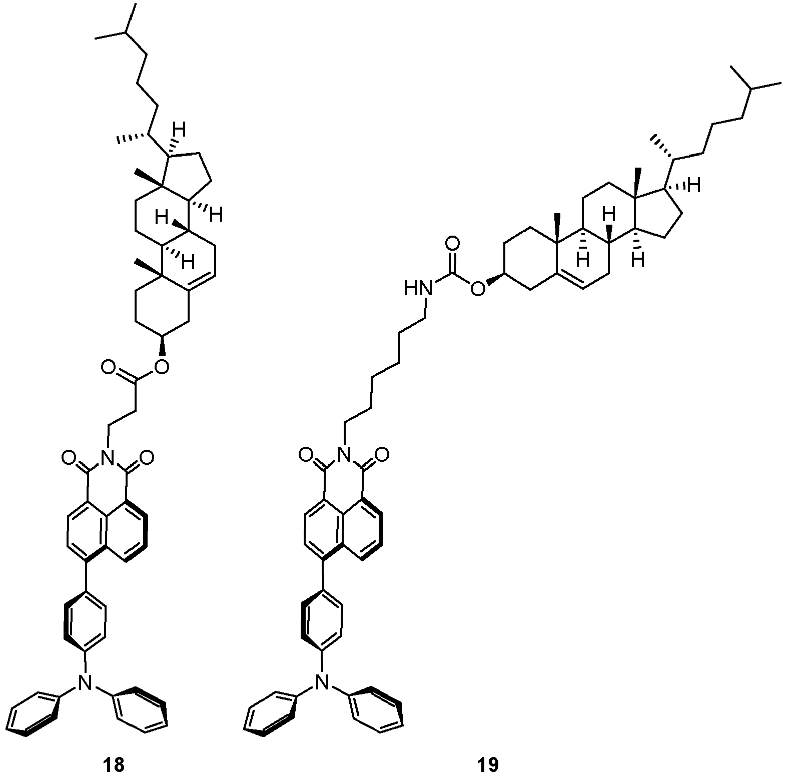

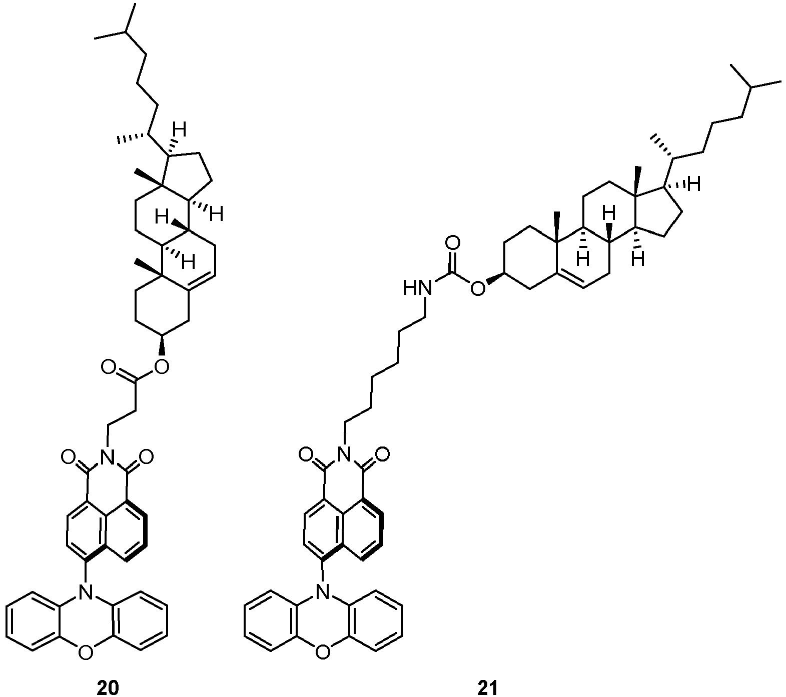

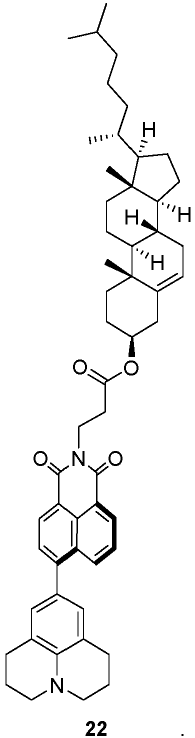

In some preferred embodiments, the luminescent compound is compound **1** or compound **2.**

The luminescent compound may be comprised in a composition further comprising an amphiphilic polymer. In the method of the first aspect, the step of contacting the cellular composition with the luminescent compound may comprise contacting the cellular composition with a composition comprising an amphiphilic polymer and the luminescent compound.

By "amphiphilic polymer" is meant a polymer having both hydrophilic regions and hydrophobic regions.

Incorporating the luminescent compound into a composition comprising an amphiphilic polymer advantageously allows the luminescent compound to be dispersed in a solvent medium in which the luminescent compound is not soluble. For example, a hydrophobic luminescent compound may be dispersed in water in this way. Therefore, use of a composition comprising an amphiphilic polymer and the luminescent compound may facilitate the uptake of the luminescent compound by cells or parts thereof in the cellular composition.

The amphiphilic polymer is suitably biocompatible. The amphiphilic polymer is suitably a copolymer, preferably a block copolymer. The block copolymer comprises at least two blocks. Suitably, at least one of the blocks is hydrophilic and at least one of the blocks is hydrophobic.

Suitably, the amphiphilic polymer is a triblock copolymer consisting of two hydrophilic blocks and one hydrophobic block. Preferably, the amphiphilic polymer is a poloxamer.

The poloxamer is a triblock copolymer having a core polypropylene glycol block and two terminal polyethylene glycol blocks. The polypropylene glycol block is hydrophobic and the polyethylene glycol blocks are hydrophilic. Any suitably poloxamer may be used, for example P188 which is available from Sigma Aldrich.

The poloxamer may have the following structure:

HO-[A]ₐ-[B]_{b}-[A]ₐ-H

wherein A is -CH₂CH₂O-, each B is independently -CH(CH₃)CH₂O- or -CH₂CH(CH₃)O-, a is at least 1, and c is at least 1.

Suitably, a is from 10 to 130, such as from 50 to 100, for example from 75 to 85, and c is from 10 to 60. such as from 15 to 45, for example from 25 to 35.

The composition comprising the amphiphilic polymer and the luminescent compound may further comprise the second solvent medium. The second solvent medium may be as defined above. Preferably, the second solvent medium is water. The presence of the second solvent medium in the composition suitably allows the luminescent compound to exhibit the second luminescent state. This may advantageously allow the composition to be identified when imaging the cellular composition.

In some preferred embodiments, the luminescent compound is comprised in a composition further comprising a poloxamer and water.

Suitably, the cellular composition may be incubated with the luminescent compound prior to imaging the cellular composition with a time-resolved spectroscopic technique. The cellular composition may be incubated with the luminescent compound for at least 30 minutes, such as at least 60 minutes, for example at least 120 minutes.

The time-resolved spectroscopic technique is suitably able to distinguish between the first luminescent state and the second luminescent state. Suitably, the time-resolved spectroscopic technique has a time resolution smaller than the difference between the first emission lifetime and the second emission lifetime. Suitably the time resolution is less than 1 ns.

Suitably, imaging the cellular composition comprises measuring a single wavelength of electromagnetic radiation, preferably a single wavelength of visible light.

Suitably, the time-resolved spectroscopic technique is fluorescence lifetime imaging (FLIM).

According to a third aspect of the present invention, there is provided a composition comprising an amphiphilic polymer and a luminescent compound that exhibits a first luminescent state having a first emission lifetime in a first chemical environment and a second luminescent state having a second emission lifetime in a second chemical environment, wherein the first emission lifetime is different to the second emission lifetime.

Suitable features of the composition, amphiphilic polymer and luminescent compound are as described in relation to the first and second aspects.

According to a fourth aspect of the present invention, there is provided a luminescent compound represented by formula (I):

A-X-B (I)

wherein A is an electron acceptor moiety comprising an aromatic group substituted with an imide group, X is a bond or a linker moiety, and B is an electron donor moiety.

Suitable features of the luminescent compound are as described in relation to the first and second aspects.

### Brief description of the Drawings

For a better understanding of the invention, and to show how example embodiments may be carried into effect, reference will now be made to the accompanying drawings in which:
Figure 1 shows the emission spectra of compound **1** recorded in varying solvent ratios of THF and water.
Figure 2 shows the emission spectra of compound **2** recorded in varying solvent ratios of THF and water.
Figure 3 shows the quantum yield of compound **2** recorded in varying solvent ratios of THF and water.
Figure 4 shows dynamic light scattering (DLS) spectra of co-assembled particles of compound **2** and the poloxamer P188.
Figure 5 shows images of the live MDA-MB 231 cells 2 hours following treatment with the P188-compound **2** particles, and a phasor plot showing phase lifetimes.

The invention will now be described with reference to the following non-limiting examples.

### Examples

### Example 1 - Synthesis and characterisation

### General methods

All solvents and chemicals were purchased from commercial sources and used without further purification. All reactions were performed using standard Schlenk techniques under an inert (N₂) atmosphere with reagent grade solvents. Flash column chromatography was performed using a Teledyne Isco CombiFlash Rf 200 automated purification system. Water was purified using a Millipore Milli-Q water purification system. ¹H and ¹³C NMR spectra were recorded on an Agilent DD2/LH spectrometer at 400 and 100 MHz, respectively. Mass-spectrometry was carried out using HPLC grade solvents. Electrospray mass spectra were determined on a Micromass LCT spectrometer and high-resolution mass spectra were carried out on a MALDI-Q-TOF-Premier (Waters Corporation, Micromass MS technologies, Manchester, UK) and high-resolution mass spectra were performed using Glu-Fib with an internal reference peak of *m*/*z* 1570.6774. Melting points were determined using an Electrothermal IA9000 digital melting point apparatus.

### Compound 1

4-bromo-1,8-naphthalic anhydride (1.0 equivalent) was reacted with *N*-octylamine (1.2 equivalents) in ethanol under reflux at 80°C for 16 hours, to give 4-bromo-*N*-octyl-1,8-naphthalimide in 80% yield. This was followed by carrying out a Suzuki-Miyaura cross-coupling reaction between 4-bromo-*N*-octyl-1,8-naphthalimide (1.0 equivalent) and 4-(9H-carbazol-9-yl)phenyl)boronic acid (1.3 equivalents) in the presence of K₂CO₃ (2.5 equivalents) and a Pd(PPh₃)₄ catalyst (5 mol%), in 1,4-dioxane/water (2:1 v/v) at 100°C for 16 hours, to give compound **1** in a yield of 63%.

Compound **1** was characterised by ¹H and ¹³C NMR spectroscopy, high resolution mass spectrometry (HRMS) and melting point analysis. In the mass spectrum, a peak was found at m/z = 551.2687, corresponding to [**1**+H]⁺. Additionally, the molecular structure of compound **1** was determined unequivocally by single crystal X-ray diffraction. The crystals were grown by slow evaporation of a DCM/hexane solution of **1.**

### Compound 2

4-bromo-1,8-naphthalic anhydride (1.0 equivalent) was reacted with *N*-octylamine (1.2 equivalents) in ethanol under reflux at 80°C for 16 hours, to give 4-bromo-*N*-octyl-1,8-naphthalimide in 80% yield. This was followed by carrying out a Suzuki-Miyaura cross-coupling reaction between 4-bromo-*N*-octyl-1,8-naphthalimide (1.0 equivalent) and 4-(9*H*-carbazol-9-yl)phenyl)boronic acid (1.3 equivalents) in the presence of K₂CO₃ (2.5 equivalents) and a Pd(PPh₃)₄ catalyst (5 mol%), in 1,4-dioxane/water (2:1 v/v) at 100°C for 16 hours, to give compound **2** in a yield of 70%.

Compound **2** was characterised by ¹H and ¹³C NMR spectroscopy, high resolution mass spectrometry (HRMS) and melting point analysis. In the mass spectrum, a peak was found at m/z = 553.2845, corresponding to [**2**+H]⁺. Despite repeated attempts to crystallise compound **2,** it could only be isolated as a glassy solid and was found not to crystallise, even at low temperatures.

### Compounds 3 to 22

Compounds **3** to **22** (as defined above) were synthesised in a similar manner to compounds **1** and **2,** using the appropriate primary amine instead of *N-*octylamine and the appropriate boronic acid in the Suzuki-Miyaura cross-coupling reaction.

### Example 2 - UV-Vis absorption and emission spectroscopy

### General methods

All samples were prepared in HPLC or spectroscopic grade solvents (THF, hexane, DMSO, MeOH, and water) with varying concentrations on the order of *µ*M. UV-visible absorption spectra were recorded at 298 K in 1 cm quartz cuvettes on a Varian Cary 50 spectrophotometer. A baseline correction was applied for all spectra. Molar absorptivity determinations were verified by linear least-squares fit of values obtained from at least three independent solutions at varying concentrations ranging from 1.00 × 10⁻⁴ to 1.00 × 10⁻⁶ M.

Steady-state emission spectra were recorded at 298 K in 1 cm quartz cuvettes using a Varian Cary Eclipse Fluorimeter with a xenon lamp light source. All samples for steady-state measurements were excited in optically dilute regimes, where the absorbance values of the chosen excitation wavelengths were less than or equal to 0.1. Time-resolved measurements were performed at 298 K with a Horiba Jobin Yvon Fluorolog FL3-22 equipped with a Fluorohub v2.0 single photon controller using the time-correlated single photon counting method (TCSPC), run in reverse mode. The sample solutions were excited at 378 nm using a PDL 800-D pulsed diode laser (NanoLED^{®}). The time distribution of the lamp pulse (<1.0 ns), also called the instrument response function, was recorded prior to lifetime measurements in a separate experiment using a scatter solution of silica nanoparticles (Ludox^{®} from Aldrich). The decays were analysed using IBH DAS6 software and the data fitted as a sum of exponentials, employing a nonlinear least-squares error minimization analysis. Emission quantum yields were determined using the optically dilute method. Individual relative quantum yield values were calculated for each solution and the values reported represent the slope value. The equation Φₛ = Φᵣ(*Aᵣ*/*Aₛ*)(*Iₛ*/*Iᵣ*)(*n*ₛ/*n*ᵣ)² was used to calculate the relative quantum yield of each of the sample, where Φᵣ is the absolute quantum yield of the reference, *n* is the refractive index of the solvent, A is the absorbance at the excitation wavelength, and / is the integrated area under the corrected emission curve. The subscripts s and r referto the sample and reference, respectively. A solution of quinine sulphate in 0.5 M H₂SO₄ (Φ_{PL} = 54.6%) was used as the external reference.

### Compounds 1 and 2

Compounds **1** and **2** were dissolved in various solvent media and the emission spectra were recorded. In each case the starting solvent was THF, and another solvent was added to form a solvent mixture with THF. The results for compound **1** are shown in Table 1 and the results for compound **2** are shown in Table 2.

**Table 1**

| **Solvent** | **Compound 1** | | | | |
|---|---|---|---|---|---|
| | **λ_{PL} (nm)^{a}** | **FWHM (cm⁻¹)^{b}** | **τ₁ [(ns) ±10%]^{c}** | **τ₂ [(ns)^{c} ±10%]** | **Φ_{PL} [(%) ±10%]^{d}** |
| THF (100%) | 531 | 3650 | 7.85 | - | 29 |
| THF (10%)/ Hexane (90%) | 464 | 3090 | 2.73 | - | 60 |
| THF (10%)/ DMSO (90%) | 622 | - | - | - | 1 |
| THF (10%)/ MeOH (90%) | 590 | - | - | - | - |
| THF (50%)/ H₂O (50%) | 600 | - | - | - | - |
| THF (10%)/ H₂O (90%) | 502 | 3190 | 8.79 (42%) | 25.0 (58%) | 16 |

| | | | | | |
|---|---|---|---|---|---|
| ^{a} λ_{exc}: 360 nm. ^{b} Full width at half maximum. ^{c} λ_{exc}: 372 nm. ^{d} Quinine sulfate was used as the reference (λ_{PL} = 54.6% in 0.5 M H₂SO₄ at 298 K). | | | | | |

**Table 2**

| **Solvent** | **Compound 2** | | | | |
|---|---|---|---|---|---|
| | **λₘₐₓ (nm)^{a}** | **FWHM (cm⁻¹)b** | **τ₁ [(ns)** ±**10%]^{c}** | **τ₂ [(ns)** ±**10%]^{c}** | **Φ_{PL} [(%)** ±1**0%]^{d}** |
| THF (100%) | 601 | 3300 | 6.71 | - | 22 |
| THF (10%)/ Hexane (90%) | 503 | 2990 | 4.08 | - | 81 |
| THF (10%)/ DMSO (90%) | 698 | - | - | - | - |
| THF (10%)/ MeOH (90%) | 663 | - | - | - | - |
| THF (50%)/ H₂O (50%) | 648 | - | - | - | - |
| THF (10%)/ H₂O (90%) | 566 | 2920 | 4.31 (57%) | 17.3 (43%) | 33 |

| | | | | | |
|---|---|---|---|---|---|
| ^{a} λ_{exc}: 360 nm. ° Full width at half maximum. ^{c} λ_{exc}: 372 nm. ^{d} Quinine sulfate was used as the reference (λ_{PL} = 54.6% in 0.5 M H₂SO₄ at 298 K). | | | | | |

Both compounds **1** and **2** displayed solvatochromic behaviour, which is a thermodynamic effect characterised by large changes in emission energies as a function of solvent polarity, driven by molecules with large dipole moments reorienting themselves with respect to the polarity of their surrounding environments. Thus, when hexane was used, large hypsochromic blueshifts occurred in the emission profiles, from yellow to deep-blue for compound **1,** and orange to sky-blue for compound **2** (λ_{PL} = 531 and 603 nm for compounds **1** and **2,** respectively, in neat THF, and 464 and 503 nm for compounds **1** and **2,** respectively, in 90:10 hexane/THF). Accordingly, the opposite solvatochromic effects are apparent when the polar solvents DMSO (λ_{PL} = 622 and 698 nm for compounds **1** and **2,** respectively, in 90:10 DMSO/THF) and MeOH (λ_{PL} = 590 and 663 nm for compounds **1** and **2,** respectively, in 90:10 MeOH/THF) were used. No significant differences were observed between aprotic DMSO and protic MeOH.

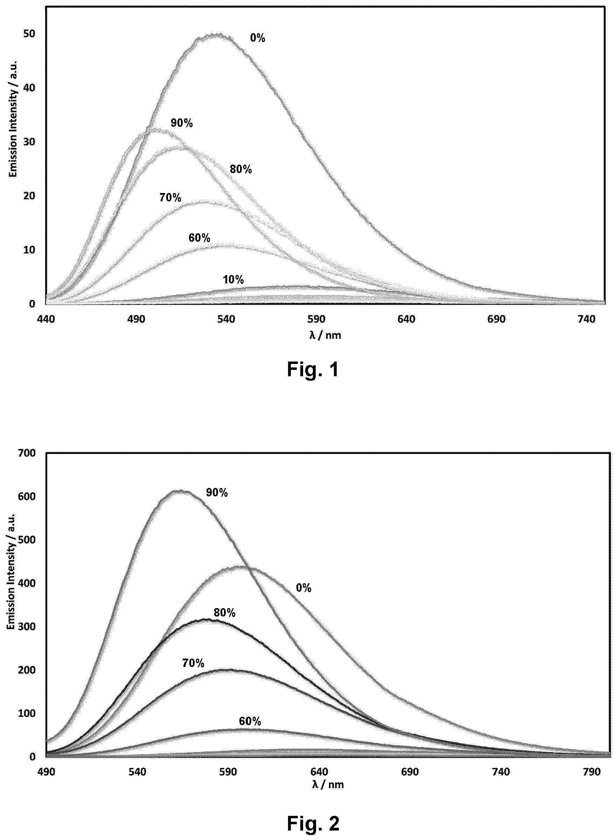

Figures 1 and 2 show the emission spectra of compounds **1** and **2** recorded in varying solvent ratios of THF and water. The percentages shown are the water fraction, e.g. 90% is 10:90 THF/water and 0% is 100% THF. Water fractions showing very little emission (20% to 50% for compound **1** and 10% to 50% for compound **2**) are unmarked.

In up to 50% water/THF fractions (λ_{PL} = 600 and 648 nm for compounds **1** and **2,** respectively, in 50:50 water/THF) the expected redshifting trend was apparent; however, at water volume fractions greater than 50%, and counterintuitively to what would be expected from the highly polar solvent environment, the emission colour underwent a significant blueshift compared to the starting state (λ_{PL} = 531 and 603 nm, respectively, for compounds **1** and **2** in 100% THF; λ_{PL} = 502 and 566 nm, respectively, for compounds **1** and **2** in 10:90 THF/water). The key process driving this anti-solvatochromic phenomenon was aggregation. Surpassing a critical water fraction (>50%) induced aggregation of the dyes, causing the chromophores to experience much less polar local environments than the bulk solvent, counteracting the expected solvatochromic trend and resulting in the THF/water emission spectra emulating the THF/hexane emission spectra, rather than THF/DMSO or THF/MeOH.

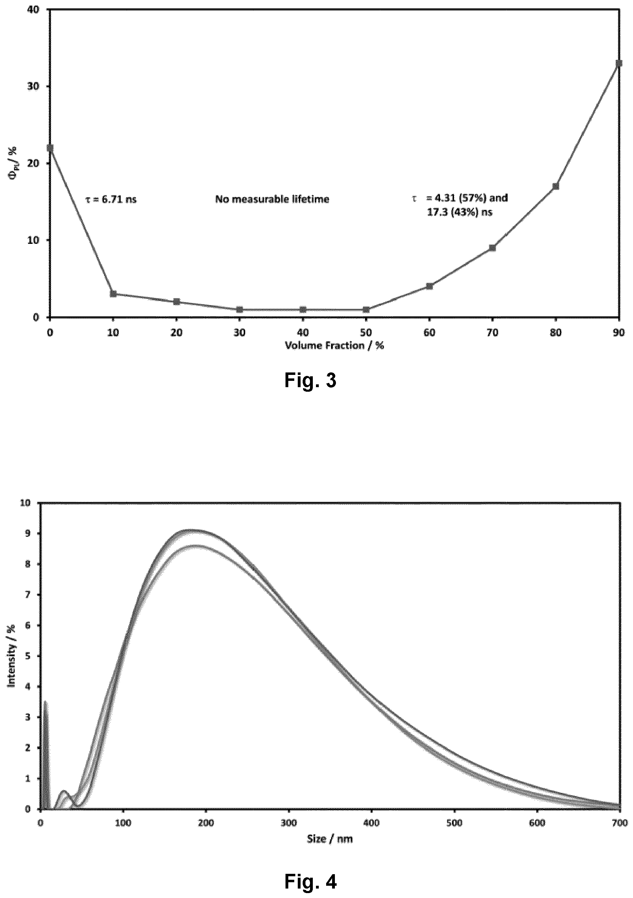

In addition to steady-state measurements, parameters (lifetimes τ, quantum yields Φ) relating to the excited state kinetics of compounds **1** and **2** were also recorded. These results are summarised in Tables 1 and 2. A plot of the quantum yield of compound **2** as a function of water volume fraction in the THF/water solvent mixture is shown in Figure 3.

The quantum yields of compounds **1** and **2** followed a trend in line with that of the emission spectra: as the emission redshifted, the quantum yields concomitantly diminished, while blueshifting the emission greatly enhanced them instead. Moving from pure THF to 90% hexane in THF significantly increased the emission intensities (Φ_{PL} = 60 and 81% for copounds **1** and **2,** respectively, in 10:90 THF/hexane) while, by contrast, even small quantities of DMSO and particularly MeOH efficiently quenched the emission, and at high volume fractions no emission was detected at all. Again, water was an outlier. Initially, water mirrored the effects in DMSO and MeOH: only a 10% water fraction resulted in near total quenching of the emission of both compounds **1** and **2** and virtually no emission is detectable in solutions up to 50% water. Above 50%, the emission was restored and continued to increase as the water content was increased, reaching a quantum yield of 16% in 90% water for compound **1.** This effect was even more pronounced for compound **2,** with a final quantum yield of 33% in 90% water - even more emissive than in pure THF (22%).

The luminescence in water is a distinct excited state process to that observed when the chromophores are dispersed in good solvent environments. In addition to enhancing the quantum yields upon addition of hexane, the lifetimes of compounds 1 and **2** shortened but remained monoexponential (τ_{PL} = 2.73 and 4.08 ns for compounds **1** and **2,** respectively). By contrast, the lifetimes of compounds **1** and **2** in 90% water in THF were biexponential and quite long for fluorescent emitters [τ_{PL} = 8.79 (42%) and 25.0 ns (58%) for compound **1;** τ_{PL} = 4.31 (57%) and 17.3 ns (43%) for compound **2**]. The short components of the decay profiles of compounds **1** and **2** were similar to the lifetime values measured for those compounds in neat THF, while the presence of longer components points toward the suppression of nonradiative decay contributions to the excited state kinetics - in particular, rotational quenching processes by the individual aromatic rings comprising the chromophores, traditionally associated with AlE molecules. This effect was more pronounced for compound **2** than for compound **1** as compound **2** is comprised of four freely rotating aromatic units, compared with just three moieties in **1.** Figure 3 shows that the luminescent state in good solvents is easily distinguishable from the luminescent state in poor solvents using time-resolved emission spectroscopy.

### Example 3 - Preparation of co-assembled particles

All samples were prepared in HPLC or spectroscopic grade solvents (THF and water) with varying concentrations on the order of µM. Dynamic Light Scattering (DLS) measurements were performed at 298 K with a Malvern Instruments Zetasizer Nano ZS provided with a 633 nm He-Ne laser (4 mW) as the light source.

Dynamic light scattering (DLS) experiments were performed on the 90% water solutions of compounds **1** and **2.** to determine the size of the particles formed by the aggregated compound. The hydrophobicity of compounds **1** and **2** drove the particles to grow significantly over time and the large particles were found to undergo poor cellular uptake.

Compound **2** and P188 were dissolved in THF and sonicated for one minute, before evaporation of the THF. Water was then added and the sample was sonicated for a further minute, before being allowed to equilibrate at room temperature for 16 hours, during which time co-assembled particles of compound **2** and P188 formed. These particles are water soluble.

P188 is a clinically approved amphiphilic poloxamer. P188 is a tri-block copolymer comprising a hydrophobic polypropylene glycol core and two peripheral hydrophilic polyethylene glycol chains. Compound **2** is entirely water insoluble and so provides a hydrophobic template about which the hydrophobic core of P188 can wrap around, directing the hydrophilic components to the exterior of the particles, rendering them water soluble.

The DLS spectrum of the co-assembled particles, measured after 16 hours in water, is shown in Figure 4. The measurements were carried out in triplicate. Two peaks are present: a small peak at 5 nm which is due to free, unbound, monomeric P188, and a much larger peak with a mean diameter of 190 nm, which was determined to be the co-assembled particles. To determine the stability of the co-assembled particles, DLS spectra were also recorded after seven days and virtually no changes were observed, with a slightly smaller mean diameter of 181 nm.

The water-solubility and stability of the co-assembled particles was expected to facilitate cell uptake compared to free compound **2.**

Photophysical measurements were also made on the co-assembled P188-compound **2** particles in water (see Table 3 below). The steady state photophysical properties resembled that of compound **2** in 10% THF in water. The emission band was moderately redshifted and the excited state lifetime was shorter but remained two-component [τ_{PL} = 2.98 (50%) and 9.76 (50%) ns for P188-compound **2** particles in water]. Thus, despite P188 presenting a more favourable environment than water for compound **2,** it is clear that compound **2** nevertheless adopts an aggregated state within the P188-compound **2** particles. As with the DLS studies, no significant changes were observed in the photophysical properties of the particles over a period of seven days.

**Table 3**

| **λ_{abs} (nm) [ε (× 10⁴ M⁻¹ cm⁻¹)** ±**10%]** | **λ_{PL} (nm)^{a}** | **τ₁ [(ns)** ±**10%]^{b}** | **τ₂ [(ns)** ±**10%]^{c}** |
|---|---|---|---|
| 308 [2.21], 356(sh) [0.66], 431 [0.93] | 579 | 2.98 (50%) | 9.76 (50%) |

| | | | |
|---|---|---|---|
| ^{a} λ_{exc}: 360 nm. ° λ_{exc}: 372 nm. | | | |

### Example 4 - Cell imaging

### Method

Cells were cultured in Dubecco's Modified Eagle Medium supplemented with 1% penicillin, 1% glutamine and 10% foetal bovine serum (FBS). MDA-MB 231 human breast cancer cells were seeded on eight chamber well slides and left for 24 hours to proliferate at 5% CO₂ and 37°C. Cells were treated with the preformed P188-compound **2** nanoparticles (diluted 1/10 in cell media to 5 µM) for 1 hour. Cells were imaged both live and following fixation. For fixing cells, removed cell media containing dye, washed with phosphate buffered saline solution (PBS) (2x200 µL), added 4% paraformaldehyde solution (200 µL) for 20 min, washed with PBS (200 µL) and then stored in PBS (200 µL).

For co-incubation experiments, cells were incubated with the preformed P188-compound **2** nanoparticles (diluted 1/10 in cell media to 5 µM) and NIR-AZA fluorophore (diluted 1/10 in cell media to 5 µM) for 1 hour. Cells were then fixed and imaged using a widefield microscope. Experiments were repeated in triplicate and the data was analysed using imaged plugin JACoP.

For FLIM studies, cells were treated with the P188-compound **2** particles for 2 hours and imaged live. Experiments were repeated and the average lifetime was calculated for 6 field of views containing both cells and background emission.

Cells were imaged using widefield and confocal microscopes. Widefield fluorescence and DIC images were acquired on an Olympus IX73 epi-fluorescent microscope fitted with an Andor iXon Ultra 888 EMCCD and controlled by MetaMorph (v7.8). Fluorescence illumination was provided by a Lumencor Spectra X light engine containing a solid-state light source. CFP: excitation filter = 435 (15) nm. Emission filter 482 (18) nm NIR: excitation filter = 640 (14) nm. Emission filter = 705 (72) nm. Images were acquired using a 60x/1.42 oil PlanApo objective (Olympus). Image processing was completed by using software imaged. Deconvolution was performed using Huygen's Professional (SVI, v15.10) software. Confocal images acquired using Leica Stellaris 8 (100X / 1.49 HC PL APO CS2).

### Results

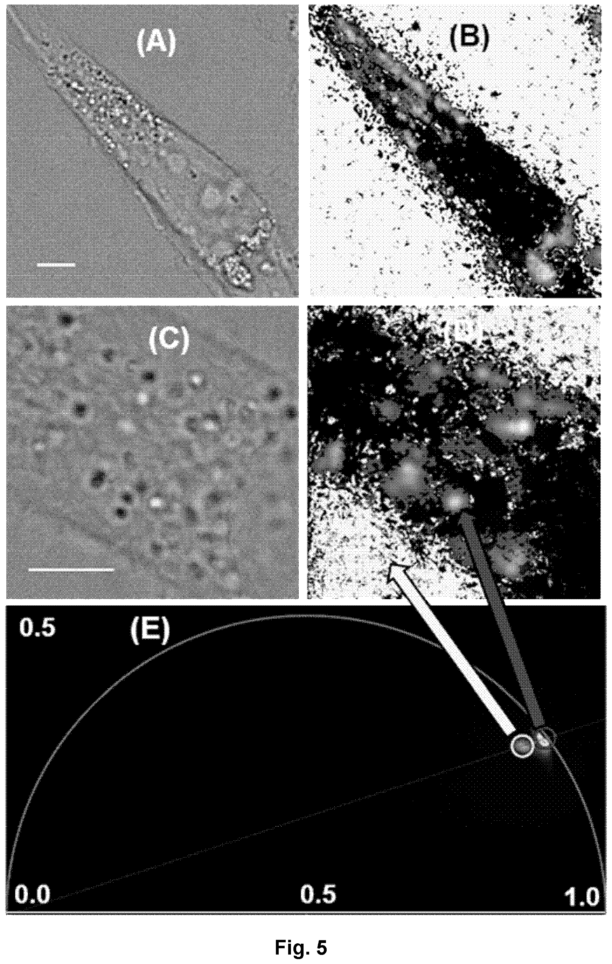

Live cell FLIM imaging was used to track the structural impact of cellular uptake on the P188-compound **2** particles. Following a 120 min incubation, FLIM imaging could evidently distinguish between intra- and extracellular compound **2** with clear differences in the measured emission lifetimes (see Figure 5). In extracellular regions, a biexponential lifetime of 3.3 (75%) and 9.0 (25%) ns was measured, which is in excellent agreement with the AlE form of compound 2 (see Tables 2 and 3). However, within specific localized areas inside the cell body a significantly different mono-exponential lifetime of 5.6 ns was measured. This value is in close agreement with lifetimes measured for the non-aggregated form of compound **2** in THF or in hexane solutions, indicating the cell can unpick the AlE state to release its monomeric form.

Figures 5(A)-(D) show images of the live MDA-MB 231 cells 2 hours following treatment with the P188-compound **2** particles. The scale bar represents 5µm. Figure 5(A) shows a brightfield cell image. Figure 5(B) shows FLIM cell imaging observing extra-cellular (3.3 and 9.0 ns, white) and intra-cellular lifetimes (5.6 ns, grey). Figure 5(C) shows an expanded brightfield cell view in which lipid droplets are visible. Figure 5(D) shows an expanded FLIM image showing different extracellular and lipid droplet lifetime regions. Figure 5(E) is a phasor plot showing two phase lifetimes.

Live cell differential interference contrast and widefield microscopy imaging confirmed the presence of distinct regions of emission throughout the cell. Super resolution radial fluctuations (SRRF) imaging and confocal laser scanning microscopy (CLSM) experiments were conducted to identify the subcellular localization of compound **2.** SRRF images showed the emission from compound **2** was localized to distinct vesicles distributed throughout the cytosol with an average diameter of 550 nm, indicative of lipid droplets. A microscope acquired emission spectrum taken of these specific organelles recorded a λₘₐₓ emission of 518 nm. This closely corresponded to the λₘₐₓ of a spectrometer recorded emission spectrum of compound **2** in triolein (triglyceride derived from glycerol and three oleic acids) which is a major component of lipid droplets. Additionally, the lifetime of compound **2** recorded in triolein (τ_{PL} = 6.19 ns) was also consistent with the FLIM imaging data discussed above.

CLSM was used to obtain a 3D z-stack view of the entire cell which provided a consistently strong indication that the vesicles were in fact lipid droplets. Final confirmation was achieved by conducting co-incubation studies of the preformed P188-compound **2** particles with a known longer wavelength emitting lipid droplet stain NIR-AZA (λ_{exc} = 640 nm, λ_{PL} = 705 nm). Statistical image analysis following 60 min co-incubation showed a high Pearson's correlation coefficient of 0.89, conclusively showing that the intracellular destination of compound **2** was the lipid droplets.

These results demonstrate that the solvent induced 'on-off-on' AlE nature of compound **2** can advantageously be used for imaging the cellular environment. In particular, due to the relatively long excited state lifetimes of compound **2** in its aggregated state, FLIM can be used to distinguish between the two different emissive states of compound **2** and map its localization within the cells. Furthermore, compound **2** may be co-assembled with the amphiphilic poloxamer P188 to facilitate cell uptake.

Although a few preferred embodiments have been shown and described, it will be appreciated by those skilled in the art that various changes and modifications might be made without departing from the scope of the invention, as defined in the appended claims.

Throughout this specification, the term "comprising" or "comprises" means including the component(s) specified but not to the exclusion of the presence of other components. The term "consisting essentially of' or "consists essentially of" means including the components specified but excluding other components except for materials present as impurities, unavoidable materials present as a result of processes used to provide the components, and components added for a purpose other than achieving the technical effect of the invention. Typically, when referring to compositions, a composition consisting essentially of a set of components will comprise less than 5% by weight, typically less than 3% by weight, more typically less than 1% by weight of non-specified components.

The term "consisting of" or "consists of' means including the components specified but excluding addition of other components.

Whenever appropriate, depending upon the context, the use of the term "comprises" or "comprising" may also be taken to encompass or include the meaning "consists essentially of' or "consisting essentially of", and may also be taken to include the meaning "consists of' or "consisting of".

The optional features set out herein may be used either individually or in combination with each other where appropriate and particularly in the combinations as set out in the accompanying claims. The optional features for each aspect or exemplary embodiment of the invention as set out herein are also to be read as applicable to any other aspect or exemplary embodiments of the invention, where appropriate. In other words, the skilled person reading this specification should consider the optional features for each exemplary embodiment of the invention as interchangeable and combinable between different exemplary embodiments.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, and drawings), and/or all of the steps of any method or process so disclosed, may be combined in any combination, except combinations where at least some of such features and/or steps are mutually exclusive.

Each feature disclosed in this specification (including any accompanying claims, and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, and drawings), or to any novel one, or any novel combination, of the steps of any method or process so disclosed.

## Claims

1. A method of imaging a cellular composition comprising the steps of:
- contacting the cellular composition with a luminescent compound that exhibits a first luminescent state having a first emission lifetime in a first chemical environment and a second luminescent state having a second emission lifetime in a second chemical environment, wherein the first emission lifetime is different to the second emission lifetime; and
- imaging the cellular composition with a time-resolved spectroscopic technique.

2. Use of a luminescent compound in combination with a time-resolved spectroscopic technique for imaging a cellular composition, wherein the compound exhibits a first luminescent state having a first emission lifetime in a first chemical environment and a second luminescent state having a second emission lifetime in a second chemical environment, wherein the first emission lifetime is different to the second emission lifetime.

3. The method or use of claim 1 or 2, wherein the time-resolved spectroscopic technique is fluorescence lifetime imaging (FLIM).

4. The method or use of any preceding claim, wherein the compound is comprised in a composition further comprising an amphiphilic polymer.

5. A composition comprising an amphiphilic polymer and a luminescent compound that exhibits a first luminescent state having a first emission lifetime in a first chemical environment and a second luminescent state having a second emission lifetime in a second chemical environment, wherein the first emission lifetime is different to the second emission lifetime.

6. The method, use or composition of any preceding claim, wherein the first chemical environment is a first solvent medium in which the luminescent compound is soluble and the second chemical environment is a second solvent medium in which the luminescent compound is insoluble.

7. The method, use or composition of any preceding claim, wherein the first chemical environment is a first solvent medium comprising a solvent selected from acetone, dioxane, isopropanol, pyridine, tetrahydrofuran, and mixtures thereof.

8. The method, use or composition of any preceding claim, wherein the second chemical environment is a second solvent medium comprising water.

9. The method, use or composition of any preceding claim, wherein the luminescent compound comprises a plurality of freely rotating aromatic groups, preferably at least three, such as at least four.

10. The method, use or composition of any preceding claim, wherein the luminescent compound is represented by formula (I):
A-X-B (I)
wherein A is an electron acceptor moiety, X is a bond or a linker moiety, and B is an electron donor moiety.

11. The method, use or composition of claim 10, wherein A has the structure: 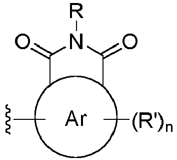
wherein R is an organic or organometallic group or a metal complex;
Ar is a monocyclic aromatic group or a polycyclic aromatic group;
n is 0, 1, 2, 3, or 4; and
each R' is independently an amino group or a hydrocarbyl group, wherein the hydrocarbyl group is optionally interrupted by an oxygen atom or a sulfur atom.

12. The method, use or composition of claim 10 or 11, wherein X is a bond or has the structure: 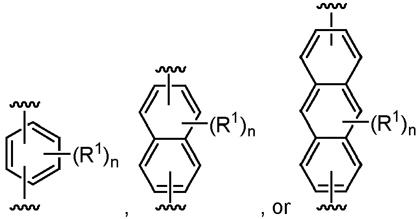 wherein n is 0, 1, 2, 3, or 4 and each R¹ is independently a hydrocarbyl group.

13. The method, use or composition of any of claims 10 to 12, wherein B is selected from: 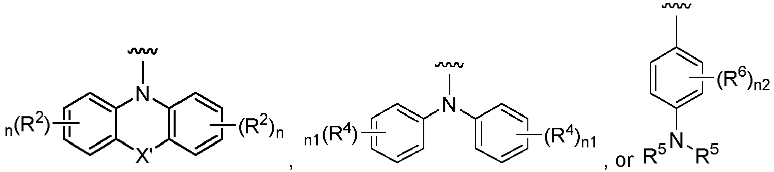
wherein each n is independently 0, 1, 2, 3, or 4;
each R² is independently an electron-donating group;
X' is a bond, oxygen, NH, NR³, sulfur, or an alkylene group;
R³ is a hydrocarbyl group;
each n1 is independently 0, 1, 2, 3, 4, or 5;
each R⁴ is independently an electron-donating group;
n2 is 0, 1, 2, 3 or 4;
each R⁵ is independently a hydrocarbyl group; and
each R⁶ is independently an electron-donating group; or
each R⁵ may independently be taken together with an R⁶ group and the intervening atoms to form a heterocycle.

14. The method, use or composition of any preceding claim, wherein the luminescent compound is represented by formula (I):
A-X-B (I)
wherein A has the structure: 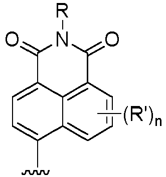
wherein R is an organic or organometallic group or a metal complex, n is 0, 1, 2, 3, or 4, and
each R' is independently a hydrocarbyl group;
X is a bond or has the structure: 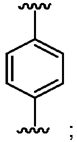 and
B is selected from: 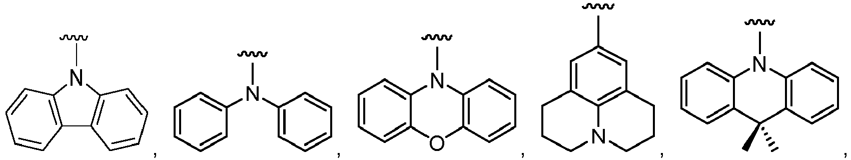 or

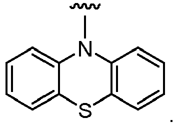

15. A luminescent compound represented by formula (I):
A-X-B (I)
wherein A is an electron acceptor moiety comprising an aromatic group substituted with an imide group, X is a bond or a linker moiety, and B is an electron donor moiety.
